# EUROPÄISCHE PATENTSCHRIFT

(11) **EP 2 793 814 B1**
(45) Veröffentlichungstag und Bekanntmachung des Hinweises auf die Patenterteilung: **13.11.2019**
(21) Anmeldenummer: 12806429.2
(22) Anmeldetag: 14.12.2012
(51) Int. Cl.: A61K 8/34, A61Q 5/00, A61K 9/00, A61K 9/06, A61K 9/107, A61K 8/40, A61K 31/16, A61K 31/045, A61Q 15/00, A61Q 17/00

(54) **WIRKSTOFFKOMBINATIONEN AUS EINER ODER MEHREREN AROMATISCHEN ALKOHOLEN UND OCTANOHYDROXAMSÄURE SOWIE KOSMETISCHE ODER DERMATOLOGISCHE ZUBEREITUNGEN, SOLCHE WIRKSTOFFKOMBINATIONEN ENTHALTEND**
ACTIVE SUBSTANCE COMBINATIONS OF ONE OR MORE AROMATIC ALCOHOLS AND OCTANOHYDROXAMIC ACIDS, AND COSMETIC OR DERMATOLOGICAL PREPARATIONS CONTAINING SUCH ACTIVE SUBSTANCE COMBINATIONS
ASSOCIATIONS DE PRINCIPES ACTIFS À BASE D'UN OU DE PLUSIEURS ALCOOLS AROMATIQUES ET D' ACIDE OCTANOHYDROXAMIQUE, AINSI QUE PRÉPARATIONS COSMÉTIQUES OU DERMATOLOGIQUES CONTENANT DE TELLES ASSOCIATIONS DE PRINCIPES ACTIFS

(30) Priorität: 19.12.2011 DE 102011088951
(43) Veröffentlichungstag der Anmeldung: 29.10.2014
(73) Patentinhaber: Beiersdorf AG, 20253 Hamburg (DE)
(72) Erfinder: FIRYN, Andreas, 21493 Schwarzenbek (DE); KÖHLER, Manuela, 22147 Hamburg (DE); TRAUPE, Bernd, 24568 Kaltenkirchen (DE); GÖKSEL, Hülya, 50733 Köln (DE)
(86) Internationale Anmeldenummer: PCT/EP2012/075532
(87) Internationale Veröffentlichungsnummer: WO 2013/092409

(56) Entgegenhaltungen:
- EP-A1- 1 671 679
- DE-A1-102005 032 592
- KR-A- 20130 061 229
- US-A1- 2009 143 489
- US-B1- 6 979 439
- DATABASE CA [Online] CHEMICAL ABSTRACTS SERVICE, COLUMBUS, OHIO, US; 2007, Ammendola, S. et al.: "Acyl hydroxamate-based biocides and their use in disinfection and preservation systems", XP002719445, gefunden im STN Database accession no. 152:310353 & IT MI20 071 800 A1 (AMMENDOLA) 18 Dezember 2007

## Beschreibung

Die vorliegende Erfindung betrifft kosmetische Wirkstoffkombinationen und Zubereitungen, solche Wirkstoffkombinationen enthaltend, sowie deren Verwendung als gegen Bakterien, Mycota und Viren wirksame Substanzen.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch insbesondere grampositive Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, dass die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine weitere Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Pilze, auch Fungi [fungus = lat. Pilz], Mycota [µυκης = grch. Pilz] oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophyten (z.B. Epidermophytie, Favus, Mikrosporie, Trichophytie), Hefemycosen (z.B. Pityriasis und andere Pityrosporum-bedingte Mykosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophyten befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche (Onychomykosen).

Ein weit verbreitetes Phänomen sind die sogenannten Kopfschuppen. Produzieren die Talgdrüsen eines Menschen zu viel Talg oder sind diese Talgdrüsen durch zu seltenes Haare waschen durch Talg verstopft, dann wird dem normalerweise harmlose Hefepilz Pitysporum ovale, heute bekannt als Malassezia Furfur, ein starkes Wachstum ermöglicht, was zum Seborrhöischen Ekzem mit Juckreiz und Schuppenbildung führt.

Eine Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung die Bildung von Kopfschuppen zu unterbinden, wobei die physiologische Hautflora keine nennenswerte Einbußen erleidet.

Ferner sind Superinfektionen der Haut durch Pilze und Bakterien nicht selten.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, dass sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nennenswerte Einbußen erleidet.

Protozoen sind parasitisch lebende Einzeller mit klar abgegrenztem Zellkern, die sich ungeschlechtlich fortpflanzen (durch Zwei- oder Vierfachteilung sowie Knospung), oder aber geschlechtlich (Gameto-, Gamonto- und Autogamie). Die Nahrungsaufnahme aus der Umgebung erfolgt durch Permeation sowie durch Pino- oder Phagozytose. Die meisten Protozoen können neben vegetativen, meist beweglichen Zustandsformen (sogenannten Trophozoiten) unter ungünstigen Umständen auch Zysten als Dauerformen ausbilden,

Je nach Fortbewegungsart und -apparat werden Protozoen in vier verschiedene Gruppen unterteilt:
(a) Mastigophora (Flagellaten mit Geißeln)
(b) Sarcodina/Rhizopoda (amöboides Bewegungsmuster durch Plasmaausstülpungen)
(c) Sporozoa (schlängelndes oder gleitendes Bewegungsmuster)
(d) Ciliata/Ciliophora (Bewimperung oder Begeißelung)

Parasitisch lebende Protozoen werden in subtropischen und tropischen Gebieten häufig durch stechende und saugende Insekten, aber auch Schmutz- und Schmierinfektion sowie durch die Nahrungskette übertragen.

Einige medizinisch und dermatologisch relevante Protozoonosen sind: Trichomoniasis (verursacht von Trichomonas vaginalis), Lamblienruhr (verursacht durch Lamblia intestinalis), viszerale sowie kutane und Schleimhaut-Leishmaniose (verursacht beispielsweise durch Leishmania donovanii, L.tropica, L.brasiliensis, L-mexicana, L.diffusa oder L. pifanoi), Trypanosmiasis (verursacht durch verschiedene Trypanosoma-Arten), Amöbenruhr und Amöbiasis (verursacht beispielsweise durch verschiedene Entamoeba-Arten, Jodamoeba butschlii oder Naegleria fowleri), Kokzidose (durch Isospora belli) und Balantidenruhr (verursacht durch Balantidium coli).

Durch Protozoonosen hervorgerufene medizinische und dermatologische Phänomene beeinträchtigen, zum Teil erheblich, das menschliche Wohlbefinden. Es besteht daher bei den betroffenen Personen ein erheblicher Bedarf, diesem Zustande abzuhelfen. Eine Aufgabe der vorliegenden Erfindung war es also, gegen Protozoen wirksame Wirkprinzipien zu finden.

Im Gegensatze zu den prokaryotischen und eukaryotischen zellulären Organismen sind Viren [Virus = lat. Gift] biologische Strukturen, welche zur Biosynthese eine Wirtszelle benötigen. Extrazelluläre Viren (auch "Virionen" genannt) bestehen aus einer ein- oder doppelsträngigen Nukleinsäuresequenz (DNS oder RNS) und einem Proteinmantel (Capsid genannt), gegebenenfalls einer zusätzlichen lipidhaltigen Hülle (Envelope) umgeben. Die Gesamtheit aus Nukleinsäure und Capsid wird auch Nucleocapsid genannt. Die Klassifikation der Viren erfolgte klassisch nach klinischen Kriterien, heutzutage allerdings zumeist nach ihrer Struktur, ihrer Morphologie, insbesondere aber nach der Nukleinsäuresequenz.

Medizinisch wichtige Virengattungen sind beispielsweise Influenzaviren (Familie der Orthomyxoviridae), Lyssaviren (z.B. Tollwut, Familie der Rhabdoviren) Enteroviren (z.B. Hepatitis-A, Familie der Picornaviridae), Hepadnaviren (z.B. Hepatitis-B, Familie der Hepadnaviridae).

Viruzide, also Viren abtötende Substanzen im eigentlichen Sinne gibt es nicht, da Viren nicht über eigenen Stoffwechsel verfügen. Es wurde aus diesem Grunde auch diskutiert, ob Viren als Lebewesen eingeordnet werden sollten. Pharmakologische Eingriffe ohne Schädigung der nicht befallenen Zellen sind jedenfalls schwierig. Mögliche Wirkmechanismen im Kampfe gegen die Viren sind in erster Linie die Störung deren Replikation, z.B. durch Blockieren der für die Replikation wichtigen Enzyme, die in der Wirtszelle vorliegen. Ferner kann das Freisetzen der viralen Nukleinsäuren in die Wirtszelle verhindert werden. Im Rahmen der hiermit vorgelegten Offenbarung wird unter Begriffen wie "antiviral" oder "gegen Viren wirksam", "viruzid" oder ähnlichen die Eigenschaft einer Substanz verstanden, einen ein- oder mehrzelligen Organismus vor schädlichen Folgen einer Virusinfektion, sei es prophylaktisch oder therapeutisch, zu schützen, ungeachtet dessen, was der tatsächliche Wirkmechanismus der Substanz im Einzelfalle sei.

Dem Stande der Technik mangelt es jedoch an gegen Viren wirksamen Substanzen, welche zudem den Wirtsorganismus nicht oder nicht in vertretbarem Maße schädigen.

Eine Aufgabe der vorliegenden Erfindung war also, diesem Übelstande abzuhelfen, also Substanzen zu finden, welche wirksam einen ein- oder mehrzelligen Organismus vor schädlichen Folgen einer Virusinfektion, sei es prophylaktisch oder therapeutisch, zu schützen.

Kosmetische Zubereitungen müssen darüber hinaus langzeitstabil gegen mikrobielle Kontamination formuliert werden.

Die mikrobielle Stabilität wird bislang durch den Zusatz an Konservierungsmitteln gelöst. Bekannte Konservierungsmittel sind zum Beispiel die als Parabene bezeichneten Verbindungen, insbesondere 4-Hydroxybenzoesäure und deren Ester, Methylparaben, Ethylparaben, Propylparaben, Isopropylparaben, Butylparaben, Isobutylparaben, Phenylparaben.

Zubereitungen ohne oder möglichst geringer Menge an Konservierungsmittel bereit zu stellen ist jedoch ein Wunsch der Konsumenten.

Eine Aufgabe der vorliegenden Erfindung war also, diesem Übelstande abzuhelfen, also Substanzen zu finden, welche wirksam gegen gram positive, gram negative, Hefen und Pilze wirken und somit ein Produkt auch über einen langen Zeitraum von mindestens 3 Jahren vor mikrobielle Kontamination zu schützen und dabei möglichst geringe Mengen an antimikrobiell wirkenden Substanzen zu verwenden.

Es wurde gefunden, und darin liegt die Lösung all dieser Aufgaben, daß Kosmetische oder dermatologische Zubereitungen mit einem Gehalt an Wirkstoffkombinationen, umfassend
a) einen oder mehrere aromatische Alkohole der allgemeinen Strukturformel 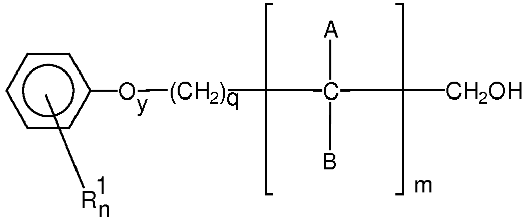 wobei R¹ darstellen kann: H, CH₃, OCH₃, NH₂, und wobei bis zu fünf gleiche oder verschiedene Reste R¹ bzw. beliebige Kombinationen gleicher und verschiedener solcher Reste innerhalb eines Moleküls auftreten können, entsprechend n = 1 - 5. Der Index m kann Werte von 1 - 10 annehmen, der Index y kann die Werte 0 oder 1 annehmen, der Index q kann Werte von 0 - 10 annehmen, A und B stellen unabhängig voneinander dar H, OH, sowie verzweigte und unverzweigte Alkylreste mit 1 - 10 Kohlenstoffatomen, und
b) Octanohydroxamsäure
den Nachteilen des Standes der Technik abhelfen.

Hydroxamsäuren sind eine Klasse chemischer Verbindungen, die als funktionelle Gruppe die Gruppierung -CO-NHOH enthalten. Ein Beispiel ist die Octanohydroxamsäure, auch Caprylohydroxamsäure genannt, welche durch folgende Struktur gekennzeichnet ist.

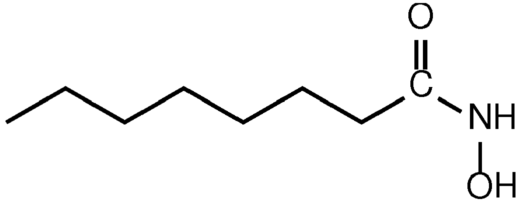

Sie ist ein bekannter Wirkstoff zur Konservierung kosmetischer Zubereitungen (z.B. WO 2009/070736 A1) Erfindungsgemäß bevorzugte aromatische Alkohole werden gewählt aus der Gruppe Benzylalkohol, Phenoxyethanol, Anisalkohol, 2-Methyl-5-phenyl-pentan-1-ol, 2-Methyl-4-phenylbutan-2-ol.

Benzylalkohol ist gekennzeichnet durch die Strukturformel

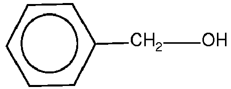

Phenoxyethanol ist gekennzeichnet durch die Strukturformel

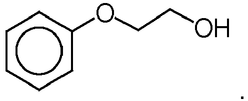

Anisalkohol ist gekennzeichnet durch die Strukturformel

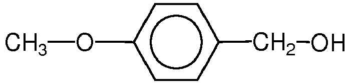

2-Methyl-5-phenyl-pentan-1-ol ist gekennzeichnet durch die Strukturformel

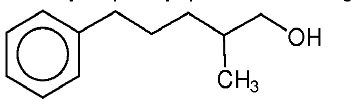

2-Methyl-4-phenylbutan-2-ol ist gekennzeichnet durch die Strukturformel

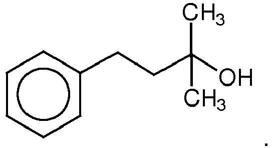

Die Dokumente US 6,979,439, EP 1 671 679 A und die US 2009 143 489 konnten nicht den Weg zur vorliegenden Erfindung weisen.

Es ist erfindungsgemäß vorteilhaft, die Gewichtsverhältnisse von Octanohydroxamsäure, einerseits zu der Gesamtmenge an einem oder mehreren aromatischen Alkoholen andererseits aus dem Bereich von 1 zu 10 bis 10 zu 1, bevorzugt von 1 zu 5 bis 5 zu 1, insbesondere bevorzugt von 1 zu 2 bis 2 zu 1 zu wählen.

Vorteilhaft werden die erfindungsgemäßen Wirkstoffkombinationen in kosmetischen oder dermatologischen Zubereitungen eingesetzt.

Erfindungsgemäße Zubereitungen enthalten vorteilhaft 0,001 bis 5,0 Gew.-% Octanohydroxamsäure, bevorzugt 0,01 bis 2,0 Gew.-%, bezogen auf das Gesamtgewicht der Zubereitungen.

Erfindungsgemäße Zubereitungen enthalten vorteilhaft 0,001 bis 5,0 Gew.-% an einem oder mehreren aromatische Alkoholen bevorzugt 0,01 bis 2,0 Gew.-%, jeweils bezogen auf das Gesamtgewicht der Zubereitungen.

Die erfindungsgemäßen Zubereitungen stellen in jeglicher Hinsicht überaus befriedigende Präparate dar.

Die kosmetischen und/oder dermatologischen Formulierungen gemäß der Erfindung können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Es hat sich in erstaunlicher Weise herausgestellt, dass die erfindungsgemäßen Wirkstoffkombinationen das Wachstum von Staphylococcus. Aureus verhindern, und dies in synergistischer Weise, also überadditiv in Bezug auf die Einzelkomponenten.

Es hat sich in erstaunlicher Weise herausgestellt, daß die erfindungsgemäß verwendeten Wirkstoffe das Wachstum von grampositiven und gramnegativen Bakterien, Mycobionten verhindert.

Die erfindungsgemäß verwendeten Wirkstoffe eignen sich darüber hinaus gut für die Verwendung als desodorierender Wirkstoff in kosmetischen Desodorantien sowie gegen unreine Haut, leichte Formen der Akne bzw. Propionibakterium acnes..

Die erfindungsgemäßen Wirkstoffe haben sich ebenfalls als besonders wirkungsvoll gegen Streptokokken erwiesen..

Schließlich hat sich herausgestellt, daß die erfindungsgemäß verwendeten Wirkstoffe den Verderb organischer Substanz, insbesondere kosmetischer und dermatologischer Zubereitungen, durch den Befall mit grampositiven und gramnegativen Bakterien, Mycobionten und Viren verhindern können, wenn sie diesen Zubereitungen zugesetzt werden Beschrieben ist auch ein Verfahren zur Bekämpfung von Mycobionten, dadurch gekennzeichnet, dass die erfindungsgemäß verwendeten Wirkstoffe gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, dass diesen organischen Produkten die erfindungsgemäß verwendeten Wirkstoffe in wirksamer Menge zugegeben werden.

Die erfindungsgemäßen Wirkstoffkombinationen sind ausgezeichnet wirksam gegen Mycobionten, insbesondere in deren Auswirkungsform der Dermatomycosen. Dabei sind die erfindungsgemäßen Wirkstoffkombinationen insbesondere befähigt, das Wachstum von Hefen, insbesondere der Pityrosporum-Arten, namentlich Pityrosporum ovale, zu verhindern.

Es hat sich ferner in überraschender Weise herausgestellt, dass die erfindungsgemäßen Wirkstoffkombinationen die Bildung von seborrhoischen Erscheinungen, insbesondere Kopfschuppen, verhindern sowie bereits vorhandene seborrhoische Erscheinungen, insbesondere Kopfschuppen, zu beseitigen.

Erfindungsgemäß sind somit auch ein Verfahren zur Bekämpfung seborrhoischer Erscheinungen, insbesondere Kopfschuppen, sowie die Prophylaxe seborrhoischer Erscheinungen, insbesondere Kopfschuppen.

Schließlich hat sich herausgestellt, dass die erfindungsgemäßen Wirkstoffkombinationen den Verderb organischer Substanz, insbesondere kosmetischer und dermatologischer Zubereitungen, durch den Befall mit Mycobionten verhindern können, wenn sie diesen Zubereitungen zugesetzt werden.

Beschrieben ist auch ein Verfahren zur Bekämpfung von Mycobionten, dadurch gekennzeichnet, dass die erfindungsgemäßen Wirkstoffkombinationen, gegebenenfalls in einem geeigneten kosmetischen oder dermatologischen Träger, mit dem durch Mycobionten kontaminierten Bereich in Kontakt gebracht werden, sowie ein Verfahren zum Schutze organischer Produkte vor dem Befall mit Mycobionten, dadurch gekennzeichnet, dass diesen organischen Produkten erfindungsgemäße Wirkstoffkombinationen in wirksamer Menge zugegeben werden.

Ferner war erstaunlich, dass die erfindungsgemäßen Wirkstoffkombinationen besonders gut wirksam sind gegen den für das Entstehen von Kopfschuppen verantwortlichen Keim Pityrosporum ovale und verwandte Keime. Eine bevorzugte Ausführungsform der vorliegenden Erfindung sind mithin gegen Kopfschuppen anzuwendende Formulierungen, beispielsweise Antischuppenshampoos.

Zur Anwendung werden die kosmetischen und/oder dermatologischen Formulierungen gemäß der Erfindung in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter, UVB und/oder mindestens einen Breitbandfilter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-ÖI (W/O) oder vom Typ Öl-in-Wasser (O/W), oder eine multiple Emulsionen, beispielsweise vom Typ Wasser-in-ÖI-in-Wasser (W/O/W), ein Gel, eine Hydrodispersion, einen festen Stift oder auch ein Aerosol darstellen.

Die kosmetischen Zubereitungen gemäß der Erfindung können kosmetische Hilfsstoffe enthalten, wie sie üblicherweise in solchen Zubereitungen verwendet werden, z.B. Konservierungsmittel, Bakterizide, Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Emulsionen sind gemäß der Erfindung vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Ganz besonders vorteilhaft enthalten die erfindungsgemäßen Zubereitungen

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe, Puder oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Duschoder Badezubereitung darstellen, enthalten vorzugsweise mindestens eine anionische, nicht-ionische oder amphotere oberflächenaktive Substanz oder Gemische daraus, Wirkstoffkombinationen gemäß der Erfindung, und Hilfsmittel, wie sie üblicherweise dafür verwendet werden.

Die folgenden Beispiele sollen die Verkörperungen der vorliegenden Erfindungen verdeutlichen. Die Angaben beziehen sich stets auf Gewichts-%, sofern nicht andere Angaben gemacht werden.

**O/W Emulsion:**

| Beispiel Nr. | **1** | **2** | **3** | **4** | **5** |
|---|---|---|---|---|---|
| Panthenol | 1,5 | 0 | 0 | 0,7 | 0 |
| Octanohydroxamsäure | 0,25 | 0,15 | 0,3 | 0,1 | 0,25 |
| Phenoxyethanol | 0.25 | 0,5 | 0 | 0 | 0 |
| Benzylalkohol | 0 | 0 | 0,5 | 0,75 | 0 |
| Paraffinum Liquidum | 0 | 3 | 3 | 0 | 0 |
| Isopropyl Palmitat | 1 | 0 | 0 | 0 | 0 |
| Caprylic/Capric Triglycerid | 0 | 3 | 3 | 0 | 1,00 |
| Cetearylalkohol | 0 | 0 | 0 | 2,5 | 4,00 |
| Cetylalkohol | 0 | 3 | 3 | 0 | 0 |
| Paraffinum Liquidum | 0 | 3 | 3 | 0 | 0 |
| Dimethicon | 0 | 3 | 3 | 0 | 0 |
| Cyclomethicon D5 | 0 | 0 | 3 | 0 | 0 |
| C12-15 Alkylbenzoat | 0 | 0 | 0 | 0 | 2,00 |
| Butyrospermum Parkii Butter | 0 | 0,5 | 0 | 0,5 | 0 |
| Dicaprylylether | 0 | 0 | 0 | 10 | 0 |
| Dicaprylylcarbonat | 2 | 0 | 0 | 0 | 0 |
| Glyceryl Stearate | 0 | 1,2 | 1,2 | 2,4 | 2,4 |
| Tapiocastärke aq. | 1 | 0 | 0 | 0 | 0 |
| Glycerin | 5 | 8 | 0 | 0 | 8 |
| 1,2-Pentadiol | 0 | 0 | 0 | 0 | 2 |
| Zitronensäure | 0,0050 | 0 | 0 | 0 | 0 |
| Natronlauge aq 45% | 0,35 | 0,01 | 0,01 | 0,25 | 1 |
| Polyglyceryl-10 Myristat | 0 | 1,8 | 0 | 0 | 0 |
| Polyglyceryl-10 Stearat | 0,7 | 0 | 1,8 | 1 | 1 |
| Carbopol 981 | 0,5 | 0,02 | 0,02 | 0 | 0 |
| Acrylic Acid/VP Crosspolymer | 0 | 0 | 0 | 0,5 | 0,75 |
| Trinatrium EDTA aq. | 0 | 1 | 1 | 0 | 1 |
| Ethylhexylmethoxycinnamat + BHT | 0 | 0 | 0 | 2 | 0 |
| Butyl Methoxydibenzoylmethane | 0 | 0 | 0 | 0 | 2 |
| Phenylbenzimidazolesulfonsäure | 0 | 0 | 0 | 0 | 2 |
| Ethylhexylsalicylat | 0 | 0 | 0 | 0 | 2 |
| Titandioxid + Trimethoxycaprylylsilan | 0 | 0 | 0 | 0,3600 | 0 |
| Octocrylen | 0 | 0 | 0 | 0 | 2 |
| Parfum | 0,10 | 0,35 | 0,35 | 0 | 0,20 |
| Wasser | ad 100 | ad 100 | ad 100 | ad 100 | ad 100 |

**Hydrodispersionsgele**

| **Beispiel Nr.** | **6** | **7** | **8** | **9** | **10** |
|---|---|---|---|---|---|
| Silikonöl, cyclisch | 8 | 10 | 0 | 3 | 0 |
| Silikonöl, linear | 0 | 0 | 0 | 0 | 3 |
| Dimethiconol | 1 | 2 | 3 | 0 | 3 |
| Ethanol | 1,0 | 5,0 | 7,5 | 1,5 | 3,0 |
| Natriumpolyacrylat | 0,2 | 0,3 | 0,3 | 0,4 | 0,10 |
| Methylpropandiol | 2 | 3 | 4 | 5 | 0 |
| Glycerin | 9 | 15 | 5 | 7,5 | 25 |
| Carbomer | 0,2 | 0,3 | 0,2 | 0,4 | 0,15 |
| Acrylate/C10-30Alkylacrylat Crosspolymer | 0,2 | 0,15 | 0,3 | 0,4 | 0,10 |
| Carrageenan (Chondrus Crispus) | 0 | 0 | 0 | 0 | 2 |
| Hamamelis Extrakt | 0,1 | 0,2 | 0,3 | 0,4 | 0 |
| Parfum | q.s. | q.s. | q.s. | q.s. | q.s. |
| Octanohydroxamsäure | 0,25 | 0,2 | 0,3 | 0,1 | 0,2 |
| Phenoxyethanol | 0.25 | 0,5 | 0 | 0 | 0,25 |
| Ethylhexylglycerin | 0 | 0 | 0 | 0 | 0,5 |
| Benzylalkohol | 0 | 0 | 0,5 | 0,75 | 0 |
| Wasser | ad 100 | ad 100 | ad 100 | ad 100 | ad 100 |

| **O/W Emulsion** | **11** | **12** | **13** | **14** | **15** |
|---|---|---|---|---|---|
| Pottasium Cetyl Phosphate | 0 | 1,0 | 0 | 0 | 0,5 |
| Sodium Stearyl Glutamate | 1,0 | | 1,0 | 0 | 0 |
| Polyglyceryl-3 Methylglycose Distearat | 0 | 0 | 0 | 2,0 | 0 |
| Cetearyl Alcohol Sodium Cetearyl Sulfate | 0 | 0 | 0 | 0 | 0 |
| Glyceryl Stearat SE | 0 | 0 | 0,60 | 0 | 0,6 |
| Polyepsilon-Lysine (ε-Polylysin) | 2 | 1 | 0,5 | 0,25 | 4 |
| Stearylalkohol | 2 | 1,5 | 0 | 0 | 0 |
| Acrylates/C₁₀₋₃₀ Alkyl Acrylat Crosspolymer | 0 | 0,2 | 0 | 0 | 0,1 |
| Xanthan Gum | 0,4 | 0 | 0,2 | 0,2 | 0,3 |
| C₁₂₋₁₅ Alkyl Benzoat | 0 | 3 | 0 | 0 | 5 |
| Dicaprylyl Carbonat | 0 | 2 | 0 | 0 | 0 |
| Myristylmyristat | 0 | 0 | 2 | 0 | 1 |
| Butylenglycol Dicaprylat/Dicaprat | 0 | 0 | 3 | 0 | 3 |
| Propylheptyl Caprylat | 5 | 0 | 5 | 2 | |
| Dicaprylyl Ether | 0 | 0 | 0 | 0 | 2 |
| Cyclopentasilxonan | 0 | 5 | 0 | 0 | 10 |
| MT Propylsilsesquioxan Wachsharz mit M= Si (C 30+)(CH₃)₂ | 0 | 3 | 0 | 0 | 1,5 |
| Dimethicone | 6 | 0 | 0 | 5 | 0 |
| Dimethiconol | 0 | 5 | 0 | 0 | 0 |
| Glycerin | 3 | 0 | 12 | 10 | 5 |
| Bis-Vinyl Dimethicon / PPG-20 Crosspolymer | 0 | 4 | 1 | 0 | 0 |
| 2-(4'-Diethylamino-2'-hydoxybenzoyl)-benzoesäu rehexylester | 3 | 5 | 1 | 0,5 | 2 |
| Octocrylen | 0 | 0 | 5 | 0 | 0 |
| Titandioxid | 0 | 0,5 | 1 | 0 | 0 |
| Phenylbenzimidazol Sulfonsäure | 0 | 0 | | 4 | 2 |
| Octylsalicylat | 0 | 0 | 5 | | |
| Polysilicon-15 | 0 | 0 | 0 | 0 | 2 |
| Ethylhexylmethoxycinnamat | 0 | 10 | 0 | 0 | 0 |
| Methylen Bis-Benzotriazolyl Tetramethylbutylphenol | 3 | 0 | 0 | 2 | 0 |
| Ethylhexyltriazon | 3 | 2 | 0 | 0 | 3 |
| Tris-Triphenyl Triazine | 0 | 0 | 2 | 0 | 0 |
| Bis-Ethylhexylphenol Methoxyphenyl Triazin | 0 | 2 | 0 | 0 | 0 |
| PVP/Hexadecen Copolymer | 0 | 0,5 | 0,1 | 0 | 0 |
| Vitamin E Acetat | 0,2 | 0,2 | 0,2 | 0,3 | 0,1 |
| Na₂H₂EDTA | 0,1 | 0,1 | 0 | 0 | 0,2 |
| Parfüm | 0,2 | 0,3 | 0,3 | 0,4 | 0,25 |
| Octanohydroxamsäure | 0 | 0 | 0,1 | 0,3 | 0,25 |
| Methylisothoazolinone | 0 | 0 | 0 | 0 | 0,2 |
| Laurylhydroxamsäure | 0,2 | 0,2 | 0 | 0 | 0 |
| Benzylalkohol | 0 | 0,5 | 0 | 0,4 | 0,5 |
| Phenoxyethanol | 0,5 | 0 | 0,5 | 0 | 3,5 |
| Natriumhydroxid | q.s. | q.s. | q.s. | q.s. | q.s. |
| Wasser | ad 100,0 | ad 100,0 | ad 100,0 | ad 100,0 | ad 100,0 |

### Wirknachweis:

Das Bakterium *S.aureus (ATCC 12000)* wurde aus einem nach EN 12353 angelegten Cryoröhrchen auf einer entsprechenden Agarplatte (TSA-Agar) ausgestrichen und bei 37°C 24 Stunden bebrütet. Von den gewachsenen Bakterien wurde eine zweite Passage angelegt, die in der Wachstumskurve eingesetzt wurde.

Eine Impföse der Bakterien wurde zu 10mL Verdünnungsmittel und 5g Glasperlen mit 4mm Durchmesser gegeben und 3 Minuten gevortext. Anschließend wurde diese Bakteriensuspension auf eine OD von 0,08 eingestellt, um eine Keimzahl von ungefähr 5^{∗}10⁷ zu gewährleisten. Diese Bakteriensuspension wurde anschließend für den Test eingesetzt.

Die Wachstumskurve wurde mittels TECAN-Pipettierroboter durchgeführt. Hierzu wurden 500µL der zu testenden Wirkstofflösungen und 500 µl der Bakteriensuspension in Eppendorfgefäßen ohne Deckel mit aufgeschweißter Folie im Cooling-Rack vorgelegt. Nach 1, 6 und 24 Stunden bei 20°C wurden aus den Eppendorfgefäßen jeweils 100µL entnommen und zu 800µL Neutralisationsmittel und 100µl Wasser pipettiert (10⁻¹-Verdünnung). Aus der 10¹-Verdünnung wurde eine 10⁻³-Verdünnung hergestellt (990µl Verdünnungsmittel und 10µl aus der 10⁻¹-Verdünnung). Diese Verdünnungen wurden mittels Spiralplatter auf Agarplatten ausplattiert.

Eine Ausnahme stellt die Kontrolle da, die nur zu Beginn abgenommen wurde. Sie wurde nochmal 1:100 verdünnt (10⁻⁵-Verdünnung). Bei den Kontrollen wurden die 10⁻³ und 10⁻⁵-Verdünnungen auf Agarplatten gebracht. Zum Schluss wurden die Platten bei 37°C 24 Stunden bebrütet und anschließend mit Hilfe des Countermaten ausgewertet.

In der Auswertung wurde der Reduktionsfaktor bestimmt. Der Reduktionsfaktor ist der Faktor, um den die Zellzahl (CFU) der Testsubstanz im Vergleich zur Kontrolle reduziert wird.

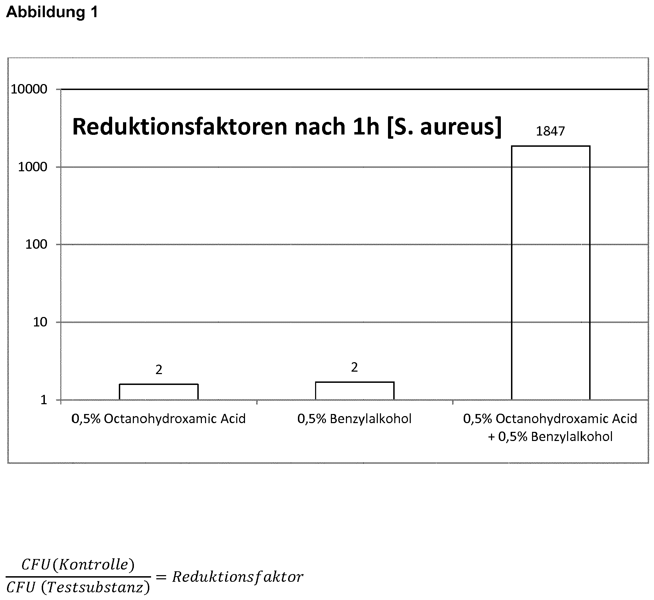

**Siehe Abbildung 1**

## Patentansprüche

1. Kosmetische oder dermatologische Zubereitungen mit einem Gehalt an Wirkstoffkombinationen, umfassend
a) einen oder mehrere aromatische Alkohole der allgemeinen Strukturformel 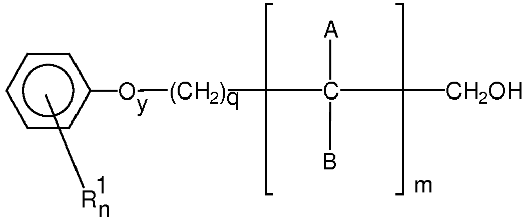 wobei R¹ darstellen kann: H, CH3, OCH3, NH2, und wobei bis zu fünf gleiche oder verschiedene Reste R¹ bzw. beliebige Kombinationen gleicher und verschiedener solcher Reste innerhalb eines Moleküls auftreten können, entsprechend n = 1 - 5. Der Index m kann Werte von 1-10 annehmen, der Index y kann die Werte 0 oder 1 annehmen, der Index q kann Werte von 0 - 10 annehmen, A und B stellen unabhängig voneinander dar H, OH, sowie verzweigte und unverzweigte Alkylreste mit 1 - 10 Kohlenstoffatomen,
und
b) Octanohydroxamsäure.

2. Zubereitungen nach Anspruch 1, **dadurch gekennzeichnet, daß** die Gewichtsverhältnisse von Octanohydroxamsäure einerseits zu der Gesamtmenge an einem oder mehreren aromatische Alkohole andererseits von 1 zu 10 bis 10 zu 1, bevorzugt von 1 zu 5 bis 5 zu 1, insbesondere bevorzugt von 1 zu 2 bis 2 zu 1 gewählt werden.

3. Zubereitungen nach Anspruch 3, **dadurch gekennzeichnet, daß** sie 0,001 bis 5,0 Gew.-% Octanohydroxamsäure enthalten, bevorzugt 0,01 bis 2,0 Gew.-%, bezogen auf das Gesamtgewicht der Zubereitungen.

4. Zubereitungen nach Anspruch 3 oder 4, **dadurch gekennzeichnet, daß** sie 0,001 bis 5,0 Gew.-% an einem oder mehreren aromatische Alkohole enthalten, bevorzugt 0,01 bis 2,0 Gew.-%, jeweils bezogen auf das Gesamtgewicht der Zubereitungen.

5. Zubereitungen nach einem der vorhergehenden Ansprüche **dadurch gekennzeichnet, daß** der oder die aromatischen Alkohole gewählt wird oder werden aus der Gruppe Benzylalkohol, Phenoxyethanol, Anisalkohol, 2-Methyl-5-phenyl-pentan-1-ol, 2-Methyl-4-phenylbutan-2-ol.

## Claims

1. Cosmetic or dermatological preparations with a content of active substance combinations comprising
a) one or more aromatic alcohols of the general structural formula 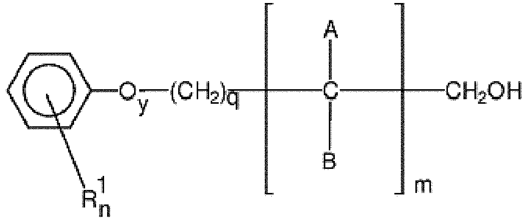 wherein R¹ can represent: H, CH₃, OCH₃, NH₂, and wherein up to five identical or different radicals R¹ and any combinations of identical and different such radicals may occur within the same molecule, corresponding to n = 1-5. Index m can take values of 1-10, index y can take the values 0 or 1, index q can take values of 0-10, A and B independently represent H, OH, and branched and also unbranched alkyl radicals having 1-10 carbon atoms,
and
b) octanohydroxamic acid.

2. Preparations according to Claim 1, **characterized in that** the weight ratios of octanohydroxamic acid to the total amount of one or more aromatic alcohols are from 1:10 to 10:1, preferably from 1:5 to 5:1, especially preferably from 1:2 to 2:1.

3. Preparations according to Claim 3, **characterized in that** they contain 0.001% to 5.0% by weight of octanohydroxamic acid, preferably 0.01% to 2.0% by weight, based on the overall weight of the preparations.

4. Preparations according to Claims 3 or 4, **characterized in that** they contain 0.001% to 5.0% by weight of one or more aromatic alcohols, preferably 0.01% to 2.0% by weight, in each case based on the overall weight of the preparations.

5. Preparations according to any of the preceding claims, **characterized in that** the aromatic alcohol(s) is/are selected from the group benzyl alcohol, phenoxyethanol, anisyl alcohol, 2-methyl-5-phenylpentan-1 -ol, 2-methyl-4-phenylbutan-2-ol.

## Revendications

1. Préparations cosmétiques ou dermatologiques ayant une teneur en combinaisons d'agents actifs, comprenant :
a) un ou plusieurs alcools aromatiques de la formule structurale générale : 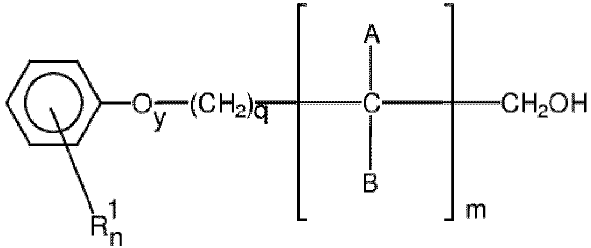 dans laquelle R¹ peut représenter : H, CH₃, OCH₃, NH₂, et dans lequelle jusqu'à cinq radicaux R¹ identiques ou différents ou des combinaisons quelconques de tels radicaux identiques et différents peuvent être présents dans une molécule, correspondant à n =1 à 5. L'indice m peut prendre des valeurs de 1 à 10, l'indice y peut prendre la valeur 0 ou 1, l'indice q peut prendre des valeurs de 0 à 10, A et B représentent indépendament l'un de l'autre H, OH, ainsi que des radicaux alkyle ramifiés et non ramifiés de 1 à 10 atomes de carbone, et
b) de l'acide octanohydroxamique.

2. Préparations selon la revendication 1, **caractérisées en ce que** les rapports en poids entre l'acide octanohydroxamique d'un côté et la quantité totale d'un ou de plusieurs alcools aromatiques d'un autre côté sont de 1 sur 10 à 10 sur 1, de préférence de 1 sur 5 à 5 sur 1, de manière particulièrement préférée de 1 sur 2 à 2 sur 1.

3. Préparations selon la revendication 3, **caractérisées en ce qu'**elles contiennent 0,001 à 5,0 % en poids d'acide octanohydroxamique, de préférence 0,01 à 2,0 % en poids, par rapport au poids total des préparations.

4. Préparations selon la revendication 3 ou 4, **caractérisées en ce qu'**elles contiennent 0,001 à 5,0 % en poids d'un ou de plusieurs alcools aromatiques, de préférence 0,01 à 2,0 % en poids, à chaque fois par rapport au poids total des préparations.

5. Préparations selon l'une quelconque des revendications précédentes, **caractérisées en ce que** le ou les alcools aromatiques sont choisis dans le groupe constitué par l'alcool benzylique, le phénoxyéthanol, l'alcool anisique, le 2-méthyl-5-phényl-pentan-1-ol, le 2-méthyl-4-phénylbutan-2-ol.
